# DEMANDE DE BREVET EUROPEEN

(11) **EP 1 810 658 A2**
(43) Date de publication de la demande: **25.07.2007**
(21) Numéro de dépôt: 06125744.0
(22) Date de dépôt: 08.12.2006
(51) Int. Cl.: A61K 8/25, A61K 8/26, A61K 8/88, A61Q 1/02, A61Q 19/02, A61Q 19/08

(54) **Composition cosmétique comprenant une dispersion aqueuse de particules colloïdales de charge minérale et des fibres**

(30) Priorité: 15.12.2005 FR 0553894
(71) Demandeur: L'Oréal, 75008 Paris (FR)
(72) Inventeur: Simonnet, Jean-Thierry, 94240 Cachan (FR)
(74) Mandataire: Kromer, Christophe

(57) **Abrégé**

L'invention concerne une composition cosmétique comprenant une dispersion aqueuse de particules colloïdales d'au moins une charge minérale, et des fibres.

Elle concerne également un procédé de traitement cosmétique de la peau, notamment d'une peau ridée, comprenant l'application sur la peau, de la composition.

## Description

La présente invention se rapporte à une composition cosmétique comprenant un agent tenseur renfermant une dispersion aqueuse de particules colloïdales d'au moins une charge minérale -en particulier de silice- et des fibres organiques, ainsi qu'un procédé cosmétique de soin de la peau.

Au cours du processus de vieillissement, une altération de la structure et des fonctions cutanées apparaît. Les principaux signes cliniques observés sont l'apparition de rides et de ridules liées à un relâchement cutané. L'homme de l'art sait qu'un tel relâchement peut être corrigé de façon immédiate par l'application d'un agent tenseur sur la peau.
A ce jour, l'utilisation de nombreux agents tenseurs pour traiter les rides est connue de l'homme de l'art. On pense en particulier aux dispersions aqueuses de particules - colloïdales ou non- d'une charge minérale, en particulier la silice, pour lisser les rides par effet tenseur , notamment décrites dans les documents US-4,819,825 , US-4,777,041, US2002/0098220, FR-2 823 113 , FR-2 659 551, WO 02/15873.

Malheureusement, les compositions contenant de tels agents tenseurs, bien que présentant un effet tenseur très satisfaisant ont l'inconvénient majeur de présenter un effet limité dans le temps.

Par conséquent, il existe un fort besoin pour des compositions cosmétiques présentant à la fois une excellente efficacité et pour lesquelles l'effet tenseur soit durable.

Or, la Demanderesse a découvert que l'inclusion de fibres dans des compositions comprenant un agent tenseur polymérique synthétique permettait d'améliorer les propriétés mécaniques du film tenseur polymérique pour lui permettre notamment de suivre les mimiques sans se fissurer, et ainsi d'améliorer sa rémanence.

La présente invention a donc pour objet une composition cosmétique comprenant dans un milieu physiologiquement acceptable au moins :
- une dispersion aqueuse de particules colloïdales d'au moins une charge minérale, lesdites particules ayant un diamètre moyen en nombre compris entre 0,1 et 100 nm, et
- des fibres organiques.

L' invention a aussi pour objet un procédé cosmétique de soin de la peau, en particulier de la peau ridée, comprenant l'application topique sur la peau d'une composition comprenant dans un milieu physiologiquement acceptable au moins :
- une dispersion aqueuse de particules colloïdales d'au moins une charge minérale, lesdites particules ayant un diamètre moyen en nombre compris entre 0,1 et 100 nm, et
- des fibres organiques.

L'utilisation de fibres en cosmétique est décrite dans de nombreux documents. On peut citer entre autre les documents EP1090626, EP1090627, EP1092424, EP1243251 et EP1262168. Toutefois, dans aucun de ces documents, on n'associe une dispersion de particules colloïdales d'au moins une charge minérale à des fibres organiques, pour corriger les signes de l'âge.

La composition et le procédé selon l'invention sont en particulier destiné à lisser la peau humaine du visage et/ou du corps et/ou à diminuer ou effacer les signes du vieillissement cutané, en particulier à réduire ou effacer les rides et/ou les ridules de la peau.
Les particules colloïdales d'au moins une charge minérale sont avantageusement présentes en quantité efficace dans la composition utilisée selon l'invention. On entend dans ce cas par « quantité efficace » la quantité au moins égale à la quantité nécessaire pour conférer à la composition un effet tenseur visible à l'oeil nu.

De même, les fibres sont de préférence présentes en quantité efficace dans la composition utilisée selon l'invention. Dans ce cas, on entend par "quantité efficace", une quantité au moins égale à la quantité nécessaire pour que cet effet tenseur soit substantiellement rémanent, c'est-à-dire encore visible au moins une heure, de préférence au moins deux heures et, mieux, au moins cinq heures, après application de la composition.

Les effets tenseur et rémanent sont notamment mesurables par scorage de photographies obtenues à différents temps après application d'un produit sur une zone présentant des rides. On peut aussi utiliser la technique dite de projection de franges qui permet une quantification fine des modifications du microrelief induites par un produit.

Par « milieu physiologiquement acceptable », on entend un milieu dénué de toxicité et compatible avec la peau et éventuellement avec ses phanères, les muqueuses et semi-muqueuses.

Dans le cadre de la présente invention, les expressions « compris entre ... et ... », « variant entre ... et ... » ou « allant de ... à ... » signifient que les bornes sont également incluses.

Comme précisé précédemment, les compositions selon l'invention comprennent au moins une dispersion de particules colloïdales d'au moins une charge minérale.
Par "particules colloïdales" au sens de la présente invention, on entend des particules ayant un diamètre moyen en nombre compris entre 0,1 et 100 nm, de préférence entre 3 et 30 nm, mieux, entre 10 et 15 nm,. Ces particules conservent les diamètres précités dans la composition les contenant, sans s'aggréger, et n'ont donc pas de propriétés épaississantes en ce sens qu'à une concentration supérieure ou égale à 15% en poids dans l'eau, , les particules colloïdales selon l'invention présentent une viscosité inférieure à 0,05 Pa.s pour un taux de cisaillement égal à 10⁻¹s, la viscosité étant mesurée à 25°C à l'aide d'un rhéomètre RheoStress RS150 de HAAKE en configuration cône-plan, le cône ayant un diamètre de 60 mm et un angle de 2°.

Cette dispersion de particules colloïdales peut être préparée selon le procédé dit "sol-gel" bien connu de l'homme du métier, à partir de sels ou d'alcoxydes des métaux correspondants solubilisés dans un solvant, tels qu'un alcool. On procède ensuite à une réaction d'hydrolyse pour former un précipité amorphe. On disperse ensuite dans l'eau avec un acide ou une base selon le pH souhaité, ce qui conduit à la peptisation du précipité et à la cristallisation. On forme de cette façon un oxyde cristallin dispersé dans l'eau.

De telles suspensions aqueuses colloïdales peuvent par exemple être préparées selon les procédés décrits dans J. Colloïd Interface Sci., 26, p. 62-69, 1968 pour SiO2, Appli. Opt. 26, 4688, 1987 pour TiO₂, Inorg. Chem., 3, 146, 1964 pour ZrO₂, Appl. Opt., 27, 3356, 1988 pour CaF₂ et MgF₂.

Ces suspensions sont préparées en utilisant des précurseurs ioniques choisis le plus souvent parmi les chlorures, les oxychlorures, les perchlorates, les nitrates, les oxynitrates ou encore les acétates ou des précurseurs moléculaires de préférence choisis parmi les alcoxydes, de formule molaire M(OR)ₙ (M représentant un métal, OR un radical alcoxy de 1 à 6 atomes de carbone et n représentant la valence du métal. Dans les méthodes décrites précédemment, le précurseur est hydrolysé ou fluoré puis polymérisé jusqu'à l'obtention d'un produit fini, insoluble dans le solvant choisi, nucléé et appelé suspension colloïdale. Dans le cas des alcoxydes, l'hydrolyse doit être rigoureusement contrôlée étant donné le caractère très hydrophile de ces dérivés organométalliques.

Un autre procédé de préparation de tels produits est décrit dans J. LIVAGE et autres, "SOL-GEL SYNTHESIS OF METAL OXYDE CLUSTERS AND COLLOIDS" (MAT. RES. SOC. SYNT. PROC., Volume 272, pages 3 à 14).

La charge minérale utilisée dans la composition selon l'invention peut notamment être choisie parmi : la silice, l'oxyde de cérium, l'oxyde de zirconium, l'alumine, le carbonate de silicium, le sulfate de baryum, le sulfate de calcium, l'oxyde de zinc, le dioxyde de titane et le platine.

Comme exemples de dispersions aqueuses de silices colloïdales utilisables selon l'invention, on peut citer celles commercialisées par la société CATALYSTS & CHEMICALS sous les dénominations commerciales COSMO S-40^{®} et COSMO S-50^{®}.

Les particules colloïdales peuvent en variante être constituées d'un oxyde mixte, en particulier d'une charge composite silice-alumine.

Par "particules de charge composite silice-alumine" au sens de la présente invention, on entend des particules constituées d'oxyde de silicium et dont la surface a été modifiée chimiquement de façon à remplacer certains au moins des atomes de silicium par des atomes d'aluminium formant au plus une couche monomoléculaire d'aluminium. Leur portion de surface recouverte d'aluminium est généralement comprise entre 1 et 100%, de préférence entre 1 et 10%, mieux, entre 4 et 6%.

Ces particules ont généralement un potentiel zêta inférieur à -20 mV, et plus préférentiellement inférieur à -25 mV, à pH 7 et à 25°C, tel que mesuré à l'aide d'un appareil DELSA 440SX de COULTER Scientific Instrument.

Elles peuvent être préparées, notamment, comme décrit dans le brevet US-2,892,797, en mélangeant un sol de silice avec un aluminate de sodium. Elles sont par ailleurs disponibles dans le commerce auprès de la société GRACE sous les référence commerciales Ludox AMX 6021^{®}, Ludox HSA^{®} et Ludox TMA^{®}.

La quantité de particules colloïdales présentes dans la composition peut varier dans une large mesure en fonction de l'effet recherché. A titre d'exemple, ces particules peuvent être présentes en une teneur en matière active allant de 0,01 à 15% en poids, et de préférence allant de 1 à 10% en poids, par rapport au poids total de la composition.

La dispersion aqueuse de particules colloïdales de charge minérale est associée dans les compositions selon l'invention à des fibres organiques.

Le facteur de forme, le titre et la morphologie des fibres sont les trois facteurs importants pour définir une fibre.

Les fibres utilisables dans les compositions selon l'invention peuvent être courtes ou longues, unitaires ou organisées par exemple tressées. Elles sont de forme généralement cylindrique contrairement aux plaquettes de forme parallélépipédique et aux particules sphériques de forme sphérique. Leur morphologie peut être quelconque et notamment de section circulaire ou polygonale (notamment carrée, triangulaire, hexagonale ou octogonale) selon l'application spécifique envisagée.

En particulier, leurs extrémités peuvent être épointées et/ou polies pour éviter de se blesser. En particulier, les fibres peuvent avoir une longueur (L) allant de 1 µm à 10 mm, de préférence de 0,1 mm à 5 mm et mieux de 0,1 mm à 1,5 mm. Leur section peut être comprise dans un cercle de diamètre (D) allant de 1 nm à 100 µm, de préférence allant de 1 µm à 50 µm et mieux de 5 µm à 40 µm. De préférence, les fibres utilisées selon la présente invention ont un facteur de forme, c'est-à-dire un rapport L/D (longueur/diamètre) allant de 3,5 à 2500, mieux de 5 à 500 et encore mieux de 5 à 150.

Le titre des fibres est souvent donné en denier ou décitex. Le denier est le poids en gramme pour 9 km de fil. De préférence, les fibres utilisées dans la composition selon l'invention ont un titre allant de 0,15 à 30 deniers, et mieux de 0,18 à 18 deniers.

Les fibres utilisables dans la composition de l'invention peuvent être des fibres organiques hydrophiles ou hydrophobes, d'origine synthétique ou naturelle.

Les fibres organiques peuvent être celles utilisées dans la fabrication des textiles et notamment des fibres de soie, de coton, de laine, de lin, de cellulose extraites notamment du bois, des légumes ou des algues, de polyamide (Nylon^{®}), de cellulose modifiée (rayonne, viscose, acétate notamment d'acétate de rayonne), de poly-p-phénylène téréphtalamide notamment de Kevlar^{®}, en acrylique notamment de polyméthacrylate de méthyle ou de poly 2-hydroxyéthyl méthacrylate, de polyoléfine et notamment de polyéthylène ou de polypropylène, d'aramide, de polytétrafluoroéthylène (notamment de Téflon^{®}), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-dessus, comme des fibres de polyamide/polyester. Comme fibres de polyuréthane, on peut citer par exemple les fibres en polymère poly(uréthane-urée), appartenant à la classe des élastanes, et notamment celles commercialisées sous la dénomination LYCRA par la société DuPont.

Les fibres organiques utilisables dans la composition selon l'invention sont de préférence choisies parmi les fibres de polyamide, les fibres de poly-p-phénylène téréphtalamide, les fibres de coton et leurs mélanges.

On peut aussi utiliser les fibres organiques synthétiques résorbables utilisées en chirurgie, comme les fibres préparées à partir d'acide glycolique et de caprolactone (Monocryl de la société Johnson & Johnson) ; les fibres synthétiques résorbables du type copolymère d'acide lactique et d'acide glycolique (Vicryl de la société Johnson & Johnson) ; les fibres de polyester téréphtalique (Ethibond de la société Johnson & Johnson). On peut aussi utiliser des mélanges des fibres citées ci-dessus.

Par ailleurs, les fibres peuvent être traitées ou non en surface, enrobées ou non. Il peut s'agir notamment de fibres enrobées et/ou fonctionnalisées. Comme fibres enrobées utilisables dans l'invention, on peut citer des fibres de polyamide enrobées de sulfure de cuivre pour un effet antistatique (par exemple R-STAT de la société Rhodia) ou un autre polymère permettant une organisation particulière des fibres (traitement de surface spécifique) ou traitement de surface induisant des effets de couleurs/hologrammes (fibre Lurex de la société Sildorex, par exemple).

Les fibres peuvent être également fonctionnalisées, c'est-à-dire être modifiées, notamment traitées en surface, de manière à avoir une fonction spécifique ou des propriétés modifiées. Cette fonctionnalisation des fibres peut être réalisée aussi bien sur les fibres que dans les fibres, et ce par toute méthode permettant d'accrocher un composé aux fibres ou de le piéger dans les cavités formées par la géométrie des fibres. Comme méthodes, on peut citer par exemple l'enduction des fibres par un actif ; la fixation de particules renfermant un actif, telles que nanocapsules ou nanosphères, sur les fibres ; l'adsorption dans les fibres ; la fixation par réaction chimique. On peut ainsi utiliser des fibres ayant des fonctionnalités particulières, par exemple des fibres anti-UV par modification avec filtres solaires chimiques ou physiques ; des fibres bactéricides ou antiseptiques par modification avec des conservateurs ou d'anti-bactériens ; des fibres colorées par modification avec des molécules colorantes ; des fibres kératolytiques ou desquamantes par modification avec des agents kératolytiques ou desquamants ; des fibres hydratantes par modification avec des agents hydratants ou des polymères rétenteurs d'eau ; les fibres parfumées par modification avec un parfum ; des fibres analgésiques ou apaisantes par modification avec un anti-inflammatoire ou un agent apaisant ; des fibres antitranspirantes par modification avec un anti-transpirant.

En particulier, on peut utiliser les fibres de polyamide commercialisées par les Etablissements P. Bonte sous le nom Polyamide 0,9 dtex 0,3 mm, ayant un diamètre moyen de 15 à 20 µm, un titre d'environ 0,9 dtex (0,81 denier) et une longueur allant de 0,3 mm à 1,5 mm. On peut aussi utiliser les fibres de poly-p-phénylène téréphtalamide de diamètre moyen de 12 µm et de longueur d'environ 1,5 mm comme celles vendues sous le nom de Kevlar Floc par la société Du Pont Fibres. Ces fibres de polyamide sont de préférence introduites dans un milieu huileux ou par voie sèche dans une poudre. On peut aussi utiliser des fibres de coton ayant un diamètre moyen de 20 µm, une longueur de 0,3 mm, et un facteur de forme de 15, telles que celles commercialisées par la société Filature de Lomme, par l'Institut Textile de France, par la société Textiles des Dunes ou par la société Velifil.

Selon leurs propriétés, les fibres utilisées selon la présente invention peuvent être introduites dans un milieu aqueux, un milieu huileux ou dans une poudre.

Les fibres organiques peuvent être présentes dans la composition selon l'invention en une quantité allant de 0,1 à 50 % en poids, par rapport au poids total de la composition, de préférence de 0,5 à 30 % en poids, mieux de 1 à 20 % en poids et encore mieux de 0,1 à 15 % (et notamment de 1 à 15 % en poids, et mieux de 2 à 15 % en poids), ou même de 0,1 à 10 % en poids (et notamment de 1 à 10 % en poids, et mieux de 2 à 10 % en poids, et préférentiellement allant de 0,1 % à 8 % en poids (et notamment de 1 % à 8 % en poids, et mieux de 2 % à 8 % en poids), et plus préférentiellement allant de 0,1 % à 5 % en poids (et notamment de 1 % à 5 % en poids, et mieux de 2 % à 5 % en poids).

Les compositions selon l'invention comprennent en plus des fibres organiques et de la dispersion aqueuse de particules colloïdales d'au moins une charge minérale, un milieu physiologiquement acceptable.
La composition de l'invention peut se présenter sous toutes les formes galéniques normalement utilisées pour une application topique sur la peau, notamment sous forme d'une solution aqueuse, hydroalcoolique ou huileuse, d'un gel aqueux ou huileux, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique, d'une émulsion directe (H/E), inverse (E/H) ou multiple (H/E/H et E/H/E). La composition utilisée selon l'invention est de préférence sous la forme d'un gel aqueux ou d'une émulsion huile-dans-eau.

Par exemple, lorsqu'elle est appliquée sur la peau, cette composition peut être plus ou moins fluide et avoir l'aspect d'une crème blanche ou colorée, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick ou d'un produit compact. Elle peut être utilisée comme produit de soin et/ou comme produit de maquillage de la peau. Elle peut par exemple être utilisée comme fond de teint.

Comme mentionné précédemment, les compositions utilisées selon l'invention peuvent comprendre une phase aqueuse.

Cette phase aqueuse peut contenir principalement de l'eau. Elle peut également comprendre un mélange d'eau et de solvant organique miscible à l'eau (miscibilité dans l'eau supérieure à 50 % en poids à 25 °C).

Cette phase aqueuse peut typiquement être présente dans une teneur supérieure ou égale à 10 %, de manière préférée à 30 %, de manière encore plus préférée à 50 %, voire à 70 % en poids par rapport au poids total de la composition.

De façon connue, la composition de l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules. Ces adjuvants ainsi que leurs concentrations doivent être tels qu'ils ne modifient pas la propriété recherchée de l'agent tenseur.

Lorsque la composition de l'invention est une émulsion, la proportion de la phase grasse peut aller de 5 à 80 % en poids, et de préférence de 5 à 50 % en poids par rapport au poids total de la composition. Les matières grasses, les émulsionnants et les coémulsionnants utilisés dans la composition sous forme d'émulsion sont choisis parmi ceux classiquement utilisés dans le domaine considéré. L'émulsionnant et le coémulsionnant sont de préférence présents, dans la composition, en une proportion allant de 0,3 à 30 % en poids, et de préférence de 0,5 à 20 % en poids par rapport au poids total de la composition.

Comme matières grasses utilisables dans l'invention, on peut citer les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires et des gommes et en particulier les gommes de silicone.

Comme émulsionnants et coémulsionnants utilisables dans l'invention, on peut citer par exemple les esters d'acide gras et de polyéthylène glycol tels que le stéarate de PEG-50 et le stéarate de PEG-40, et les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Lorsqu'elle est utilisée pour atténuer les signes cutanés du vieillissement, la composition utilisée selon l'invention peut comprendre, comme actifs, au moins un composé choisi parmi : les agents desquamants et/ou hydratants ; les agents dépigmentants ; les agents anti-glycation; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents myorelaxants ; les agents anti-pollution et/ou anti-radicalaires ; et leurs mélanges.

Des exemples de tels actifs sont : le rétinol et ses dérivés tels que le palmitate de rétinyle ; l'acide ascorbique et ses dérivés tels que l'ascorbyl phosphate de magnésium et le glucoside d'ascorbyle ; le tocophérol et ses dérivés tels que l'acétate de tocophéryle ; l'acide nicotinique et ses précurseurs tels que la nicotinamide ; l'ubiquinone ; le glutathion et ses précurseurs tels que l'acide L-2-oxothiazolidine-4-carboxylique ; les extraits de plantes et notamment les extraits de criste marine et de feuille d'olivier, ainsi que les protéines végétales et leurs hydrolysats tels que les hydrolysats de protéines de riz ou de soja ; les extraits d'algues et en particulier de laminaires ; les extraits bactériens ; les sapogénines telles que la diosgénine et les extraits de Dioscorées, en particulier de Wild Yam, en contenant ; les α-hydroxyacides ; les β-hydroxyacides, tels que l'acide salicylique et l'acide n-octanoyl-5-salicylique ; les oligopeptides et pseudodipeptides et leurs dérivés acylés, en particulier l'acide {2-[acetyl-(3-trifluoromethyl-phenyl)-amino]-3-methyl-butyrylamino} acétique et les lipopeptides commercialisés par la société SEDERMA sous les dénominations commerciales Matrixyl 500 et Matrixyl 3000 ; le lycopène ; les sels de manganèse et de magnésium, en particulier les gluconates ; et leurs mélanges.

En outre, on peut aussi associer aux agents tenseurs utilisés selon l'invention d'autres composés connus de l'homme du métier comme agents tenseurs, notamment les protéines végétales, les polysaccharides d'origine végétale sous forme ou non de microgels, les amidons et les silicates mixtes.

Lorsqu'elle est destinée à lisser la peau du corps, en particulier comme composition amincissante et/ou raffermissante, la composition utilisée selon l'invention peut en variante comprendre un ou plusieurs composés drainants, lipolytiques, désinfiltrants, amincissants, raffermissants, anti-glycants et/ou vaso-protecteurs.

Des exemples de tels composés peuvent être choisis parmi : les inhibiteurs de phosphodiestérase tels que la caféine et la théobromine ; le mannuronate de monométhylsilanetriol ; les extraits de thé, de café, de guarana, de maté, ou de cola (Cola Nitida) ; les extraits de Ginkgo biloba ; les extraits d'escine ; les extraits de Dioscorées contenant de la diosgénine ; les extraits d'algues et en particulier de Laminaria Digitata ; et leurs mélanges.

En cas d'incompatibilité, les actifs indiqués ci-dessus peuvent être incorporés dans des sphérules, notamment des vésicules ioniques ou non-ioniques et/ou des nanoparticules (nanocapsules et/ou nanosphères), de manière à les isoler les uns des autres dans la composition.

### EXEMPLES

### Exemple 1 : compositions anti-rides

Les exemples ci-après de compositions sont donnés à titre d'illustration et sans caractère limitatif. Dans ces exemples, les composés portent leur nom INCI.

Les exemples 1A et 1B suivants sont préparés selon le mode opératoire ci-dessous.

La phase B (à l'exception de l'ammonium polyacryldiméthyltauramide) est chauffée à environ 75 °C. L'ammonium polyacryldiméthyltauramide est incorporé au reste de la phase B qui est agitée jusqu'à obtention d'un gel homogène.

La phase A est également chauffée à environ 75 °C et une émulsion est réalisée en incorporant cette phase A dans la phase B.

Lorsque cette émulsion a une température de 40-45 °C, on y incorpore la phase C et le cas échéant (exemple 2B) la phase D. L'émulsion est agitée et l'agitation est maintenue jusqu'à refroidissement complet.

Exemple 1A : Emulsion huile dans eau (comparatif).

| Phase | Nom | Concentration (% massique) |
|---|---|---|
| A | Mélange de stéarate de glycéryle et de stéarate de polyéthylèneglycol-100 (ARLACEL 165FL de UNIQEMA) | 2,00 |
| | Mélange de tartrate de dimyristyle, d'alcool cétéarylique, d'alcool laurylique oxyéthyléné (25OE) oxypropyléné (25 OE) (Cosmacol PSE de SASOL) | 1,50 |
| | Cyclohexasiloxane | 10,00 |
| | Alcool Stearylique | 1,00 |
| B | Eau | qsp 100 |
| | Conservateurs | 0,75 |
| | Sel Pentasodique d'éethylène diamine tétraméthylène phosphonate | 0,05 |
| | Ammonium Polyacryldimethyltauramide (HOSTACERINE AMPS de CLARIANT): | 0,40 |
| | gomme de Xanthane (RHODICARE S de RHODIA) | 0,20 |
| C | Dispersion aqueuse de silice colloïdale (COSMO S40 de Catalysts & Chemicals. | 17,10 |

Exemple 1 B : composition cosmétique anti-rides - émulsion huile dans eau

| Phase | Nom | Concentration (% massique) |
|---|---|---|
| A | Mélange de stéarate de glycéryle et de stéarate de polyéthylèneglycol-100 (ARLACEL 165FL de UNIQEMA) | 2,00 |
| | Mélange de tartrate de dimyristyle, d'alcool cétéarylique, d'alcool laurylique oxyéthyléné (25OE) oxypropyléné (25 OE) (Cosmacol PSE de SASOL) | 1,50 |
| | Cyclohexasiloxane | 10,00 |
| | Alcool Stearylique | 1,00 |
| B | Eau | qsp 100 |
| | Conservateurs | 0,75 |
| | Sel Pentasodique d'éethylène diamine tétraméthylène phosphonate | 0,05 |
| | Ammonium Polyacryldimethyltauramide (HOSTACERINE AMPS de CLARIANT): | 0,40 |
| | gomme de Xanthane (RHODICARE S de RHODIA) | 0,20 |
| C | Dispersion aqueuse de silice colloïdale (COSMO S40 de Catalysts & Chemicals. | 17,10 |
| D | Fibres de polyamide (Nylon 66 - Pulpe polyamide de 12185 de Paul BONTE) | 2,50 |

### Exemple 2 : Mise en évidence de l'amélioration des propriétés de rémanence des formules selon l'invention

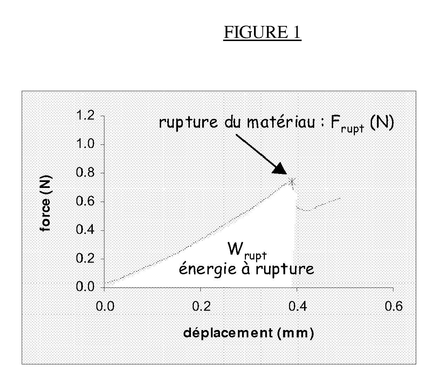

L'essai consiste à solliciter en compression jusqu'à rupture un matériau (en l'occurrence les crèmes anti-rides des exemples 1A et 1B) déposé en surface d'une mousse souple et déformable. L'utilisation de ce support en mousse permet d'imposer une importante déformation au matériau déposé en surface, et donc une quantification de sa résistance à la rupture. La sollicitation mécanique en compression est exercée à l'aide d'un poinçon cylindrique de diamètre 1 mm ; la vitesse de déplacement du poinçon étant de 0,1 mm/s. L'essai est réalisé à l'aide d'un analyseur de texture TA-XT2i commercialisé par la société Stable Micro System. Il est ainsi obtenu une courbe force F (en N) en fonction du déplacement d (en mm) à partir de laquelle il est possible de déterminer le point de rupture du matériau. La figure 1 en annexe présente un tel exemple de courbe de force en fonction du déplacement.

Le paramètre Wrupt (l'énergie à rupture en J/m²) est retenu pour quantifier la résistance à la rupture du matériau. Le paramètre correspondant à la surface sous la courbe F=f(d) / surface du poinçon.

Le substrat est constitué d'une mousse néoprène de 13 mm d'épaisseur. Le matériau (composition antirides) est déposé sur ce substrat de façon à obtenir après un séchage de 24 h un film d'une épaisseur de 15 à 30 µm. Les dépôts ont été réalisés à l'aide d'un tireur de film déposant 650 µm humide.

Les résultats obtenus sont les suivants :

| Matériau | Wᵣᵤₚₜ (J/m²) |
|---|---|
| Exemple 1A (comparatif) | 15 ± 4 |
| Exemple 1B | 171 ± 246 |

On constate que les fibres de polyamide utilisées dans la composition de l'exemple 1 B ont un rôle renforçateur, ce rôle de renfort s'illustrant par une augmentation de l'énergie à la rupture. Il en résulte un effet tenseur plus rémanent de la composition de l'exemple 1B. En effet, si l'effet tenseur est lié à la formation d'un dépôt rigide, on comprend que la pérennité de cet effet est liée à la résistance mécanique de ce dépôt.

Il a par ailleurs été vérifié sur un panel de 6 femmes que la composition de l'exemple 1 B présentait un effet tenseur satisfaisant.

## Revendications

1. Composition cosmétique comprenant, dans un milieu physiologiquement acceptable, au moins :
- une dispersion aqueuse de particules colloïdales d'au moins une charge minérale, lesdites particules ayant un diamètre moyen en nombre compris entre 0,1 et 100 nm, et
- des fibres organiques.

2. Composition selon la revendication précédente, **caractérisée en ce que** ladite charge inorganique est choisie parmi : la silice, l'oxyde de cérium, l'oxyde de zirconium, l'alumine, le carbonate de silicium, le sulfate de baryum, le sulfate de calcium, l'oxyde de zinc, le dioxyde de titane et le platine.

3. Composition selon la revendication 1, **caractérisée en ce que** ladite charge inorganique est un oxyde mixte.

4. Composition selon la revendication précédente, **caractérisée en ce que** ladite charge inorganique est une charge composite silice-alumine.

5. Composition selon la revendication précédente, **caractérisée en ce que** la surface desdites particules qui est recouverte d'aluminium est comprise entre 4 et 6 %.

6. Composition selon la revendication 4 ou 5, **caractérisée en ce que** lesdites particules ont un potentiel zêta inférieur à -25 mV, à pH 7 et à 25°C.

7. Composition selon la revendication précédente, **caractérisée en ce que** lesdites particules ont un diamètre moyen en nombre compris entre 3 et 30 nm.

8. Composition selon l'une quelconque des revendications précédentes, **caractérisée en ce que** les particules colloïdales de charge minérale sont présentes en une teneur en matière active allant de 0,01 à 15 % en poids, et de préférence allant de 1 à 10 % en poids, par rapport au poids total de la composition.

9. Composition selon l'une quelconque des revendications précédentes, **caractérisée en ce que** les fibres ont un titre allant de 0,15 à 30 deniers, et mieux de 0,18 à 18 deniers.

10. Composition selon l'une quelconque des revendications précédentes, **caractérisée en ce que** les fibres ont un facteur de forme allant de 3,5 à 2500, mieux de 5 à 500 et encore mieux de 5 à 150.

11. Composition selon l'une quelconque des revendications précédentes, **caractérisée en ce que** les fibres ont une longueur allant de 1 µm à 10 mm, de préférence de 0,1 mm à 5 mm et mieux de 0,1 mm à 1,5 mm.

12. Composition selon l'une quelconque des revendications précédentes, **caractérisée en ce que** les fibres ont une section pouvant être comprise dans un cercle de diamètre allant de 1 nm à 100 µm, de préférence allant de 1 nm à 50 µm et mieux de 5 µm à 40 µm.

13. Composition selon l'une quelconque des revendications précédentes, **caractérisée en ce que** les fibres sont choisies parmi les fibres de soie, de coton, de laine, de lin, de cellulose, de polyamide, de cellulose modifiée (rayonne, viscose, acétate notamment d'acétate de rayonne), de poly-p-phénylène téréphtalamide, en acrylique notamment de polyméthacrylate de méthyle ou de poly 2-hydroxyéthyl méthacrylate, de polyoléfine et notamment de polyéthylène ou de polypropylène, d'aramide, de polytétrafluoroéthylène, de collagène insoluble, de polyesters, de polychlorure de vinyle ou de vinylidène, d'alcool polyvinylique, de polyacrylonitrile, de chitosane, de polyuréthane, de polyéthylène phtalate, de copolymère d'acide lactique et d'acide glycolique, de polyester téréphtalique, les fibres formées d'un mélange de polymères tels que ceux mentionnés ci-dessus, comme les fibres de polyamide/polyester, les fibres préparées à partir d'acide glycolique et de caprolactone et/ou un de leurs mélanges, et leurs mélanges.

14. Composition selon la revendication précédente, **caractérisée en ce que** les fibres sont choisies parmi les fibres de polyamide, les fibres de poly-p-phénylène téréphtalamide, les fibres de coton et/ou leurs mélanges.

15. Composition selon l'une quelconque des revendications précédentes, **caractérisée en ce que** les fibres sont des fibres traitées et/ou enrobées.

16. Composition selon l'une quelconque des revendications précédentes, **caractérisée en ce que** les fibres sont présentes en une quantité allant de 0,1 à 50 % en poids, de préférence de 0,5 à 30 % en poids, mieux de 1 à 20 % en poids et encore mieux de 2 à 15 %, ou même de 2 à 10 % en poids par rapport au poids total de la composition.

17. Composition selon l'une quelconque des revendications précédentes, **caractérisée en ce qu'**elle se présente sous la forme sous la forme d'un sérum, d'une lotion, d'une émulsion directe (H/E), inverse (E/H) ou multiple (H/E/H et E/H/E), d'un stick ou d'un produit compact.

18. Composition selon l'une quelconque des revendications précédentes, **caractérisée en ce que** la composition est une composition anti-rides.

19. Composition selon l'une quelconque des revendications précédentes, **caractérisée en ce que** la composition est un fond de teint.

20. Procédé de traitement cosmétique de la peau, notamment d'une peau ridée comprenant l'application sur la peau, d'une composition telle que définie dans l'une quelconque des revendications 1 à 19.

21. Procédé selon la revendication précédente, **caractérisée en ce qu'**il est destiné à réduire ou effacer les rides et/ou les ridules de la peau et/ou lisser la peau.
